# EUROPÄISCHE PATENTSCHRIFT

(11) **EP 0 681 868 B1**
(45) Veröffentlichungstag und Bekanntmachung des Hinweises auf die Patenterteilung: **30.07.2003**
(21) Anmeldenummer: 95107123.2
(22) Anmeldetag: 11.05.1995
(51) Int. Cl.: B01J 21/18, C07C 209/36, C07D 239/545, B01J 23/44, B01J 37/02

(54) **Kohlenstoffhaltige Katalysatorträger und Verfahren zu deren Herstellung**
Carbon containing catalyst support and process for its preparation
Support de catalyseur contenant du carbone et son procédé de préparation

(30) Priorität: 13.05.1994 DE 4416903; 16.09.1994 DE 4433023; 08.05.1995 DE 19516273
(43) Veröffentlichungstag der Anmeldung: 15.11.1995
(73) Patentinhaber: KataLeuna GmbH Catalysts, 06236 Leuna (DE)
(72) Erfinder: Schödel, Rainer, Dr., D-06122 Halle (DE); Geyer, Reinhard, Dr., D-06126 Halle (DE); Birke, Peter, Prof. Dr., D-06179 Langenbogen (DE); Keck, Michael, D-06667 Reichardtswerben (DE)
(74) Vertreter: Schrell, Andreas, Dr.

(56) Entgegenhaltungen:
- EP-A- 0 069 514
- EP-A- 0 316 159
- EP-A- 0 595 124
- GB-A- 1 156 532
- NL-A- 8 600 646
- US-A- 4 956 325
- US-A- 5 053 574
- 'Römpp's Chemie Lexikon', 1992 9.Auflage * Seite 3587 - Seite 3588 *

## Beschreibung

Die Erfindung betrifft neue Katalysatorträger und Verfahren zu deren Herstellung sowie deren Verwendung. Die Katalysatorträger sind insbesondere für die Herstellung von edelmetallhaltigen Trägerkatalysatoren für die Feinchemikalienindustrie geeignet.

Als Träger für Katalysatoren für die Feinchemikalienindustrie werden vorwiegend Aktivkohlen eingesetzt. Als Aktivkomponenten werden insbesondere die Metalle Palladium, Platin, Rhodium und Ruthenium auf die Aktivkohleoberfläche aufgebracht (US 5158668, DD 296228, US 4956325). Der Einsatz von Aktivkohlen erfolgt allerdings nicht problemlos. Die Hauptschwierigkeit besteht in der Reproduzierbarkeit der Aktivkohleherstellung. Diese ungenügende Reproduzierbarkeit hat wiederum Folgen für die Wirksamkeit der Metall-Trägerkatalysatoren. Schwankende Textureigenschaften und variierende Zusammensetzung der Trägeroberfläche beeinflussen die Metalldispersität und das Ausmaß der Wechselwirkung zwischen Träger und Metall signifikant. Die Folgen sind Selektivitäts- und Aktivitätsschwankungen, also Eigenschaften, die insbesondere bei der Herstellung von Feinchemikalien nicht erwünscht sind. Weiterhin besitzen zahlreiche Aktivkohlen einen hohen Anteil an Mikroporen, die das Edelmetall aufnehmen, das dann in vielen Fällen aus sterischen Gründen nicht an der Reaktion teilnehmen kann.

Die Herstellung makroporöser Aktivkohlen führt zu mechanisch instabilen Produkten, die als Katalysatorträgermaterial nicht verwendbar sind.

Ein weiterer Nachteil ist, daß die meisten dieser Aktivkohlen in vielen Fällen vor dem Aufbringen der Edelmetalle noch oxidierend, z. B. mit H₂O₂, HNO₃ oder HClO behandelt werden müssen.

Aus US 5053574 sind Katalysatoren aus porösem Aluminiumoxid-Partikeln (Al₂O₃) beschrieben, die durch Verkokung von Kohlenwasserstoffen an ihrer Oberfläche vorzugsweise bei Temperaturen ab 250°C entstehen.

Aus GB 1156532 sind Katalysatoren bekannt, die durch Beschichtung von porösen anorganischen Trägermaterialien mit Produkten der Pyrolyse aromatischer, gesättigter oder ungesättigter aliphatischer Kohlenwasserstoffe, die vorzugsweise keine Heteroatome enthalten, hergestellt werden.

In NL 8600646 werden ebenfalls organische Trägermaterialien (Al₂O₃/SiO₂) durch Pyrolyse einer Vielzahl organischer Verbindungen ab einer Temperatur von 350°C mit einer Kohlenstoffschicht überzogen.

Die in diesen Druckschriften offenbarten Katalysatoren weisen jedoch eine Reihe von Nachteilen auf, wozu eine verbesserungsfähige Effizienz der katalysierten Hydrierungsreaktion zählt. Der vorliegenden Erfindung liegt daher die Aufgabe zugrunde, kohlenstoffhaltige Katalysatorträger mit reproduzierbarer Textur und Oberflächenchemie zu entwickeln, die ohne Vorbehandlung mit Edelmetallionen beladen werden können und auf Basis dieser Träger hochaktive, chemisch resistente und mechanisch stabile Hydrierkatalysatoren herzustellen.

Diese Aufgabe wird erfindungsgemäß dadurch gelöst, daß die Oberfläche eines anorganischen Trägermaterials mit definierter Porenstruktur mit polymeren und/oder polykondensierten Kohlenstoffverbindungen überzogen wird.

Als anorganische Trägermaterialien werden insbesondere Al₂O₃, SiO₂, TiO₂ . Al₂O₃, Tone, Zeolithe, ZrO₂ oder natürliche Silikate und/oder beliebige Gemische dieser Stoffe eingesetzt, wobei deren Oberflächen im Bereich von 1 bis 900 m²/g liegen können.

Diese Trägermaterialien können auch chemisch modifiziert vorliegen, wie z. B. durch Behandeln mit Fluorid-, Chlorid-, Sulfat- oder Phosphatverbindungen. Durch Mischen verschiedener oder auch gleicher Trägermaterialien unterschiedlicher Textur und nachfolgendes Überschichten mit Koks können so erfindungsgemäß beliebige, bzw. gewünschte Porengrößenverteilungen in den kohlenstoffhaltigen Katalysatorträgern erzielt werden.

Der Kohlenstoffgehalt der erfindungsgemäßen Katalysatorträger liegt je nach Einsatzgebiet zwischen 0,1 bis 30 Masse-%.

Die Herstellung der kohlenstoffhaltigen Katalysatorträger erfolgt erfindungsgemäß entweder durch Tränken des anorganischen Trägermaterials mit organischen Verbindungen und nachfolgender Temperung unter nichtoxidierenden Bedingungen bei Temperaturen von 100 bis 1000 °C und/oder durch Behandeln der anorganischen Trägermaterialien mit Gemischen aus nichtoxidierenden Gasen und organischen Verbindungen bei Temperaturen von 150 bis 1000°C. Als nichtoxidierendes Gas kommt insbesondere Stickstoff zum Einsatz.

Zweckmäßigerweise setzt man die anorganischen Trägermaterialien vor der erfindungsgemäßen Behandlung gleich in der Form, d. h. als Stränge, Kugeln, Pillen oder dgl. ein, in der sie dann als Katalysator zum Einsatz kommen sollen.

Die organischen Verbindungen, die als Ausgangsstoffe für die Ausbildung der Kohlenstoffschicht eingesetzt werden, sind gesättigte und/oder ungesättigte Kohlenwasserstoffverbindungen sowohl mit Heteroatomen als auch ohne Heteroatome. So können z. B. Gemische von Methylcyclopentan und Benzoesäure oder Butene mit Phenolen umgewandelt werden. Der Anteil der Verbindungen mit Heteroatomen bestimmt die Heterogenität der kohlenstoffhaltigen Oberflächenschicht. Um den Anforderungen an den Umweltschutz gerecht zu werden, ist es erforderlich, die Abgase, die bei der Verkokung entstehen, am Reaktorausgang katalytisch zu verbrennen.

Gegenüber den herkömmlichen Aktivkohle-Trägern besitzen die erfindungsgemäßen kohlenstoffhaltigen Katalysatorträger außer ihrer Reproduzierbarkeit ihrer Herstellung noch folgende Vorzüge:
- beliebige Einstellung von Porengrößenverteilungen und
- gezielte Modifizierung der Natur der Oberfläche bei der Synthese der Katalysatorträger.

Die erfindungsgemäßen kohlenstoffhaltigen Katalysatorträger werden mit Vorteil für die Herstellung neuer, hochaktiver, chemisch resistenter und mechanisch stabiler Katalysatoren für die Hydrierung von Nitroaromaten verwendet.

Erfindungsgemäß bestehen diese Katalysatoren aus Palladium mit und ohne Promotoren auf einem Träger, dessen anorganische Grundsubstanz durch Umsetzen von organischen Verbindungen mit funktionellen Gruppen in nichtoxidierender Atmosphäre mit einer funktionalisierten Kohlenstoffschicht bedeckt ist, deren Masseanteil 1 bis 20 Masse-% beträgt.

Es war überraschend, daß Pd auf einer mit einer funktionalisierten Kohlenstoffschicht bedeckten anorganischen Trägeroberfläche eine außerordentlich hohe Nitroaromatenhydrieraktivität besitzt. Dabei kann der Katalysator entweder nur Pd enthalten, oder Pd promotiert durch Zusätze von Elementen der 6. bis 8. Nebengruppe des PSE. Bevorzugter Promotor ist Platin.

Wesentlich für den erfindungsgemäßen Katalysator ist, daß die funktionalisierte Kohlenstoffschicht Ionenaustauscheigenschaften besitzt. Solche Schichten können z. B. durch thermisches Zersetzen eines Gemisches aus Phenol und Cyclohexanon im Stickstoffstrom an einer anorganischen Trägeroberfläche (SiO₂, Al₂O₃, SiO₂.Al₂O₃ u.a.) bei Temperaturen von 600 bis 750 °C erzeugt werden.

Der Anteil der Kohlenstoffschicht an der Trägermasse sollte 1 bis 20 Masse-% betragen. Als besonders vorteilhaft haben sich Kohlenstoffgehalte von 4 bis 10 Masse-% erwiesen.

Der Gehalt an auf dem Träger aufgebrachten Metall liegt im Bereich von 0,1 bis 15 Masse-%, vorzugsweise liegen die Metallgehalte im Bereich von 1 bis 4 Masse-%.

Von Bedeutung für die hohe Effektivität des erfindungsgemäßen Katalysators ist auch eine hochdisperse Verteilung der Metalle auf der Trägeroberfläche. Der Anteil der Oberflächen-Metallatome an der Gesamtzahl der Metallatome muß mindestens 50 % betragen, vorzugsweise liegen mindestens 80 % der Metallatome als Oberflächenatome vor.

Wichtig für den erfindungsgemäßen Katalysator ist außerdem, daß die Metalle im wesentlichen in für die Reaktionspartner leicht zugänglichen Poren angeordnet sind. Deshalb liegt der Anteil des Porenvolumens mit Porenradien kleiner als 2 nm am Gesamtporenvolumen unterhalb von 5 %.

Für den Einsatz der Katalysatoren in Batch-Verfahren ist eine gute Sedimentation oder Filtrierbarkeit erforderlich. Diesen Anforderungen wird der erfindungsgemäße Katalysator dadurch gerecht, daß die Teilchengrößen im Bereich von 5 bis 20 µm liegen.

Die Pd-haltigen Katalysatoren können, wie bereits ausgeführt, durch Zusätze der Elemente der 6. bis 8. Nebengruppe des PSE promotiert sein. Als besonders günstig haben sich geringe Zusätze von 0,01 bis 0,2 Masse-% Pt erwiesen. Erfolgt der Einsatz des Katalysators für die Hydrierung von halogenierten Nitroaromaten, so ist der Zusatz von Inhibitoren zum Reaktionsgemisch oder der Einsatz von sulfidiertem Pt auf den C-beschichteten anorganischen Trägern zweckmäßig, um das Dehalogenieren zu unterdrücken.

Die Herstellung der erfindungsgemäßen Katalysatoren erfolgt zunächst mit der oben beschriebenen Trägerherstellung.

Die organischen Verbindungen, die als Ausgangsstoffe für die Ausbildung der Kohlenstoffschicht eingesetzt werden, sind die bereits genannten.

Die auf diese Weise hergestellten kohlenstoffbeschichteten anorganischen Träger werden dann in einer ammoniakalischen Lösung der Metallsalze bei pH-Werten von 10 bis 14, vorzugsweise von 10,5 bis 11,5, suspendiert und gerührt. Zur annähernden Konstanthaltung des pH-Wertes ist während der Reaktion Ammoniakwasser zuzugeben oder eine Pufferlösung einzusetzen. Das Volumenverhältnis von Katalysatorträger zur Lösung soll 1 : 10 bis 1 : 2000 betragen; besonders vorteilhaft sind Verhältnisse von 1 : 15 bis 1 : 60. Nach einer Reaktionszeit von 1 bis 40 h, vorzugsweise von 12 bis 24 h, wird der Katalysator abfiltriert, bei Temperaturen von 80 bis 100 °C getrocknet und anschließend reduziert. Für die Reduktion können verschiedene Reduktionsmittel eingesetzt werden. Zu hochaktiven Katalysatoren führt insbesondere die Reduktion mit Wasserstoff bei Temperaturen von 100 bis 400 °C.

Die erfindungsgemäßen Katalysatoren zeichnen sich durch
- eine hohe Hydrieraktivität,
- eine hohe Aktivitätsstabilität,
- sehr gutes Sedimentationsvermögen und Filtrierbarkeit sowie
- ein gutes Handling infolge der guten Rieselfähigkeit aus.

Weiterhin werden die erfindungsgemäßen kohlenstoffhaltigen Katalysatorträger mit Vorteil für die Herstellung neuer, hochaktiver, chemisch resistenter und mechanisch stabiler Katalysatoren für die Hydrierung der Nitrosogruppe im 1,3-Dimethyl-4-Amino-5-Nitrosouracil zum entsprechenden Diamin verwendet. Das Hydrierprodukt aus dieser Reaktion dient als Ausgangsstoff für die technische Coffeinsynthese.

Erfindungsgemäß wird bei dieser Hydrierung mit Wasserstoff oder Wasserstoff, der durch katalytischen Zerfall von Ameisensäure gebildet wird, bei 20 bis 120 °C und 0,1 bis 10 MPa ein Katalysator eingesetzt, der 0,5 bis 7 Masse-% Pd, vorzugsweise 2 bis 4 Masse-% Pd, auf einem Träger enthält, dessen anorganische Grundsubstanz durch Umsetzen organischer Verbindungen in nichtoxidierender Atmosphäre mit einer funktionalisierten Kohlenstoffschicht bedeckt ist, mit
a) einem Anteil der Palladiumoberflächenatome an der Gesamtzahl der Palladiumatome von mindestens 75 %, vorzugsweise mindestens 90 %,
b) einem Kohlenstoffgehalt des Trägers von 1 bis 15 Masse-%, vorzugsweise 3 bis 8 Masse-%, und
c) einem Anteil des Porenvolumens mit Porenradien < 1,5 nm am Gesamtporenvolumen geringer als 10 %.

Es war überraschend, daß Pd auf einem mit einer funktionalisierten Kohlenstoffschicht bedeckten anorganischen Trägeroberfläche eine außerordentlich hohe Aktivität in der Hydrierung von 1,3-Dimethyl-4-Amino-5-Nitrosouracil sowohl mit gasförmigem Wasserstoff als auch mit Wasserstoff, der durch am Palladium erfolgten katalytischen Ameisensäurezerfall im Reaktionsgemisch gebildet wird, aufweist.

Wesentlich für die Hydrierung ist, daß der zur Herstellung des Katalysators verwendete mit Kohlenstoff beschichtete anorganische Träger Ionenaustauscheigenschaften besitzt. Solche Kohlenstoffschichten können z.B. durch thermisches Zersetzen eines Gemisches von Anilin und Cyclohexen oder Phenol und Cyclohexanon im Stickstoffstrom an einer anorganischen Trägeroberfläche (ZrO₂.Al₂O₃, SiO₂.Al₂O₃, SiO₂.Al₂O₃, Al₂O₃ u.a.) erzeugt werden. Eine weitere Variante der Herstellung solcher Trägerschichten ist das Verkoken des anorganischen Trägers mit Methylcyclopentan und eine nachfolgende Behandlung mit H₂O₂, NaOCl oder anderen oxidierend wirkenden Reagenzien.

Der Anteil der Kohlenstoffschicht an der Masse des Katalysatorträgers liegt im Bereich von 1 bis 15 Masse-%. Als besonders vorteilhaft haben sich Kohlenstoffgehalte von 3 bis 8 Masse-% erwiesen.

Der Gehalt an auf dem Träger aufgebrachten Palladium beträgt 0,5 bis 7 Masse-%, vorzugsweise liegen die Palladiumgehalte im Bereich von 2 bis 4 Masse-%.

Wichtig für die Effektivität des Hydrierverfahrens ist der Einsatz eines erfindungsgemäßen Katalysators mit einer sehr hohen Metalldispersität. Der Anteil der Oberflächen-Palladiumatome an der Gesamtzahl der Palladiumatome muß mindestens 75 % betragen, vorzugsweise liegen mindestens 90 % der Palladiumatome als Oberflächenatome vor.

Von Bedeutung ist weiterhin, daß das Palladium im wesentlichen in für die Ameisensäure oder den Wasserstoff leicht zugänglichen Poren angeordnet ist. Deshalb sollte der Anteil des Porenvolumens mit Porenradien kleiner als 1,5 nm am Gesamtporenvolumen unterhalb von 10 % liegen.

Die Reaktionsführung des Verfahrens in seiner einfachsten Form besteht in der Suspendierung der 1,3-Dimethyl-4-Amino-5-Nitrosouracils und des Katalysators in Wasser sowie in der Zuführung von Wasserstoff unter intensivem Rühren. Wird Ameisensäure als Wasserstoffquelle verwendet, so wird der wäßrigen Suspension von 1,3-Dimethyl-4-Amino-5-Nitrosouracil Ameisensäure zugesetzt und intensiv gerührt.
Der Katalysator wird zweckmäßigerweise in einer Partikelgrößenfraktion von 5 bis 15 µm eingesetzt. Damit ist eine hohe Wirksamkeit und eine gute Filtrierbarkeit des Katalysators gewährleistet.
Die katalytische Hydrierung kann von Raumtemperatur bis 120 °C und Reaktionsdrücken von 0,1 bis 10 MPa vorgenommen werden, wenn mit gasförmigem Wasserstoff reduziert wird. Beim Einsatz von Ameisensäure als Wasserstoffquelle sind Temperaturen von 20 bis 80 °C und Normaldruck zweckmäßig.

Das Wesen der Erfindung wird durch die nachfolgenden Beispiele näher erläutert.

Beispiele für die Herstellung der erfindungsgemäßen Katalysatorträger:

### Beispiel 1

500 g eines γ -Al₂O₃ in Form von Kugeln mit Durchmessern von 1,9 bis 2,3 mm und mit einer Oberfläche von 286 m²/g werden nach vorheriger thermischer Aktivierung bei 500 °C an der Luft bei 50 °C mit einem Gemisch von 5 Masse-% o-Xylol und 95 Masse-% Phenol entsprechend Wasseraufnahme getränkt. Nachfolgend wird der auf diese Weise behandelte Träger im N₂-Strom (1 l/h) auf 100 °C hochgeheizt und 2 h bei dieser Temperatur belassen. Anschließend erfolgt das Aufheizen des Trägers mit einer Aufheizrate von 2 °C/min auf 650 °C. Bei dieser Temperatur wird der Träger noch 3 h belassen und anschließend unter N₂-Spülung auf Raumtemperatur abgekühlt. Der Katalysatorträger enthält 6,8 Masse-% Kohlenstoff in der Oberflächenschicht. Seine Oberfläche beträgt 271 m²/g.

### Beispiel 2

50 g eines pulverförmigen Al₂O₃ . SiO₂ mit 40 Masse-% SiO₂ und einer Oberfläche von 326 m²/g werden nach Aktivierung bei 630 °C im N₂-Strom (11 l/h) mit einem Gemisch aus N₂ (11 l/h), Methylcyclopentan (0,6 g/h), Ethanol (1,9 g/h) und Benzoesäure (2,1 g/h) bei dieser Temperatur über einen Zeitraum von 4,5 h behandelt und danach auf Raumtemperatur unter N₂-Strömung abgekühlt. Der mit Kohlenstoff beschichtete Katalysatorträger enthält 13,8 Masse-% Kohlenstoff und besitzt eine Oberfläche von 312 m²/g.

### Beispiel 3

500 g eines strangförmigen Trägers mit 2 mm Durchmesser, bestehend aus 60 Masse-% γ -Al₂O₃ und 40 Masse-% α-Al₂O₃, mit einer Oberfläche von 138 m²/g werden an der Luft bei 550 °C über einen Zeitraum von 2 h aktiviert. Nach Abkühlen auf 30 °C werden die Stränge mit einem Gemisch aus 5 Masse-% m-Xylol, 30 Masse-% Cyclohexanon und 65 Masse-% Phenol entsprechend Wasseraufnahme getränkt, im Stickstoffstrom (1 l/h) zunächst auf 100 °C hochgeheizt, 1,5 h bei dieser Temperatur belassen und nachfolgend mit einer Aufheizrate von 1,5 °C/min auf 680 °C hochgeheizt, 2 h bei dieser Temperatur behandelt und danach auf Raumtemperatur unter Stickstoffspülung abgekühlt. Der mit Kohlenstoff beschichtete Katalysatorträger enthält 7,4 Masse-% Kohlenstoff und besitzt eine Oberfläche von 131 m²/g und Porenmaxima bei 3,8 und 72 nm Porenradius.

### Beispiel 4

100 g eines strangförmigen Trägers mit 2 mm Durchmesser, bestehend aus 70 Masse-% γ-Al₂O₃, 10 Masse-% ZrO₂, 10 Masse-% TiO₂, 5 Masse-% CeHY-Zeolith (Modul 5,1) und 5 Masse-% Al₂O_{3 .} SiO₂ (40 Masse-% SiO₂) mit einer Oberfläche von 209 m²/g werden im Stickstoffstrom auf 635 °C hochgeheizt und bei dieser Temperatur mit einem Gemisch aus Stickstoff (10,5 l/h), Cyclohexen (3,2 g/h), Cyclohexanon (2,6 g/h) und Phenol (3,3 g/h) über einen Zeitraum von 3 h behandelt. Danach wird unter Stickstoffströmung auf Raumtemperatur abgekühlt. Der mit Kohlenstoff beschichtete Katalysatorträger besitzt einen Kohlenstoffgehalt von 18,1 Masse-% und eine Oberfläche von 201 m²/g.

Beispiele für die Anwendung der erfindungsgemäßen Katalysatorträger für Pd-haltige Katalysatoren zur Hydrierung von Nitroaromaten:

### Beispiel 1

50 g eines SiO₂-Trägers mit einer BET-Oberfläche von 327 m²/g und einer Korngröße von 8 bis 16 µm werden mit einer Belastung von 500 v/g.h mit einer Aufheizgeschwindigkeit von 5 °C/min im Stickstoffstrom auf 710 °C hochgeheizt und 1 h bei dieser Temperatur belassen. Nachfolgend werden über einen Zeitraum von 3 h 5 ml/h eines Phenol-Cyclohexanon-Gemisches (50 Masse-% Phenol) in den Stickstoffstrom dosiert und durch die Trägerschüttung geführt. Anschließend wird der Träger im Stickstoffstrom auf Raumtemperatur abgekühlt, ausgebaut und in ein Reaktionsgefäß überführt, das zuvor mit 1500 ml einer ammoniakalischen PdCl₂-Lösung beschickt wurde. Die Katalysatorträgerzugabe erfolgt unter Rühren. Der pH-Wert wird während der Reaktion konstant bei 10,8 bis 11 gehalten. Die Pd-Menge in der Lösung beträgt 1,07 g/l.

Nach einer Reaktionszeit von 20 h wird der Katalysator abfiltriert, mit 1 l destilliertem Wasser gewaschen und anschliessend 10 h bei 80 °C getrocknet. Danach wird der Katalysator über einen Zeitraum von 2 h bei 200 °C mit Wasserstoff bei einer Belastung von 500 v/g.h reduziert.

Der Katalysator wird durch folgende Eigenschaften charakterisiert:

| | |
|---|---|
| BET-Oberfläche | 289 m²/g |
| C-Gehalt | 6,8 Masse-% |
| %-Anteil des Porenvolumens mit Porenradien < 2 nm | 0 |
| F-Wert ¹⁾ | 0,87 |
| Pd-Gehalt | 2,97 Masse-% |

| | |
|---|---|
| ¹⁾ F-Wert: Verhältnis der Zahl der Metalloberflächenatome zur Gesamtzahl der Metallatome | |

Die Zahl der Oberflächenmetallatome wird durch CO-Impuls-Chemisorption bei 0 °C ermittelt. Dabei wird die Annahme getroffen, daß 1 Pd-Atom ein CO-Molekül chemisorbiert.

### Beispiel 2

Analog wie Beispiel 1, nur enthält die Lösung nicht 1,07 g Pd/l, sondern 0,96 Pd/l und 0,036 g Pt/l. Der Katalysator besitzt folgende Eigenschaften:

| | |
|---|---|
| BET-Oberfläche | 291 m²/g |
| C-Gehalt | 6,9 Masse-% |
| %-Anteil des Porenvolumens mit Porenradien < 2 nm | 0 |
| F-Wert | 0,89 |
| Pd-Gehalt | 2,7 Masse-% |
| Pt-Gehalt | 0,1 Masse-% |

Die Hydrieraktivität ist aus der Tabelle ersichtlich.

### Beispiel 3

50 g eines Al₂O₃.SiO₂-Trägers mit 40 Masse-% SiO₂ und einer BET-Oberfläche von 237 m²/g sowie einer Korngröße von 5 bis 11 µm werden mit einer Belastung von 500 v/g.h im Stickstoffstrom mit einer Aufheizgeschwindigkeit von 3 °C/min auf 740 °C hochgeheizt und 1 h bei dieser Temperatur belassen. Danach werden über einen Zeitraum von 2,5 h 5 ml/g eines Phenol-Cyclohexanon-Gemisches (70 Masse-% Phenol) in den Stickstoffstrom dosiert und durch die Trägerschüttung geleitet. Nach Abkühlen im Stickstoffstrom auf Raumtemperatur wird der Träger unter Rühren in 1000 ml einer ammoniakalischen PdCl₂-Lösung mit einem Pd-Gehalt von 1,25 g Pd/l eingetragen. Der pH-Wert wird während der Reaktion konstant bei 10,9 bis 11,2 gehalten. Nach 15-stündigem Rühren wird der Katalysator durch Filtration von der Lösung getrennt, mit destilliertem Wasser chloridfrei gewaschen und anschließend 10 h bei 80 °C getrocknet. Der getrocknete Katalysator wird 2 h bei 175 °C mit Wasserstoff bei einer Belastung von 500 v/g.h reduziert.

Der Katalysator besitzt folgende Charakteristika:

| | |
|---|---|
| BET-Oberfläche | 221 m²/g |
| C-Gehalt | 7,6 Masse-% |
| %-Anteil des Porenvolumens mit Porenradien < 2 nm | 1,2 |
| F-Wert | 0,84 |
| Pd-Gehalt | 2,49 Masse-% |

Die Hydrieraktivität ist aus der Tabelle ersichtlich.

Die Aktivitäten der Katalysatoren wurden in der Hydrierung von Nitrobenzol gemessen. Hierzu wurde eine Schüttelapparatur eingesetzt, die unter einem geringen Überdruck von 20 kPa arbeitet. 25 mg Katalysator werden mit 10 ml Wasser, 15 ml Ethanol und 25 ml Anilin in das Reaktionsgefäß überführt und auf 80 °C aufgeheizt. Anschließend werden 400 µl Nitrobenzol zudosiert, die Apparatur mit Wasserstoff gespült und die Schüttelvorrichtung in Betrieb genommen. Die Schüttelgeschwindigkeit beträgt 300 Schüttelbewegungen pro Minute. Der bei der Hydrierung verbrauchte Wasserstoff wird einem Manostaten entnommen. Aus dem Wasserstoffverbrauch wird die Reaktionsgeschwindigkeit in ml H₂ pro g Katalysator und Minute berechnet.

Die auf diese Weise ermittelten Hydrieraktivitäten der mit den erfindungsgemäßen Trägern hergestellten Katalysatoren sind in der Tabelle zusammengestellt.

Weiterhin sind Aktivitätswerte für Vergleichskatalysatoren angeführt.

Die Gegenüberstellung der Meßdaten belegt die Vorzüge der erfindungsgemäßen Katalysatoren.

**Tabelle**

| Katalysator | Hydrieraktivität in ml H₂/min.g Kat. |
|---|---|
| Beispiel 1 | 624 |
| Beispiel 2 | 643 |
| Beispiel 3 | 634 |
| Produkt Nr. 009708 (Heraeus) 5 % Pd/C | 528 |
| Produkt-Nr. 009713 5 % Pd/C | 441 |

Beispiele für die Anwendung der erfindungsgemäßen Katalysatorträger für Pd-haltige Katalysatoren zur Hydrierung von 1,3-Dimethyl-4-Amino-5-Nitrosouracil:

### Beispiel 1

1 g 1,3-Dimethyl-4-Amino-5-Nitrosouracil, 50 mg Katalysator und 50 ml Wasser werden in ein temperierbares und mit Wasserstoff gespültes Reaktionsgefäß überführt und auf die Reaktionstemperatur von 60 °C erwärmt. Nach Erreichen der Reaktionstemperatur wird der Reaktor mit einem Wasserstoff enthaltenden Manostaten verbunden. Anschließend wird durch Anstellen der Schüttelapparatur die Reaktion gestartet. Bei einer Schüttelgeschwindigkeit von 305 Schüttelbewegungen pro Minute und einem Druck von 0,102 MPa wird der Wasserstoffverbrauch gemessen. Aus dem Wasserstoffverbrauch wird die Reaktionsgeschwindigkeit in ml Wasserstoff pro g Katalysator und Minute berechnet.

Als Katalysatoren werden eingesetzt:
Katalysator A: (Vergleichskatalysator) 5 Masse-% Pd/Aktivkohle (Aldrich, Cat.No. 20.568-0)
Katalysator B: (Vergleichskatalysator) 5 Masse-% Pd/C (Heraeus, 009708)
Katalysator C: (Vergleichskatalysator) 5 Masse-% Pd/C (Degussa, E 101 NO/W)
Katalysator D: (erfindungsgemäß) 3,7 Masse-% Pd/C-SiO₂
   - C-Gehalt: 8,1 Masse-%
   - %-Anteil des Porenvolumens mit Porenradien < 1,5 nm: 7,5 %
   - F-Wert¹⁾: 0,91
Katalysator E: (erfindungsgemäß) 4,0 Masse-% Pd/C-Al₂O₃
   - C-Gehalt: 7,3 Masse-%
   - %-Anteil des Porenvolumens mit Porenradien < 1,5 nm: 4 %
   - F-Wert: 0,9
Katalysator F: (erfindungsgemäß) 3,5 Masse-% Pd/C-SiO₂
   - C-Gehalt: 6,9 Masse-%
   - %-Anteil des Porenvolumens mit Porenradien < 1,5 nm: 7,8 %
   - F-Wert: 0,93

¹⁾ F-Wert: Verhältnis der Zahl der Metalloberflächenatome zur Gesamtzahl der Metallatome

Die Zahl der Oberflächenatome wird durch CO-Impuls-Chemisorption bei 0 °C ermittelt. Dabei wird die Annahme getroffen, daß 1 Palladiumatom ein CO-Molekül chemisorbiert.

Die gemessenen Hydrieraktivitäten der verschiedenen Verfahren sind in der Tabelle 1 als Hydriergeschwindigkeiten zusammengestellt. Ein Vergleich der Meßdaten verdeutlicht die Vorzüge des Einsatzes der erfindungsgemäßen Katalysatoren.

**Tabelle 1**

| Katalysator | Hydriergeschwindigkeit in ml H₂/g Kat.min |
|---|---|
| A | 138,6 |
| B | 156,7 |
| C | 36,9 |
| D | 171,7 |
| E | 189,4 |
| F | 197,8 |

### Beispiel 2

1 g 1,3-Dimethyl-4-Amino-5-Nitrosouracil, 50 mg Katalysator, 50 ml Wasser und 1,5 g Ameisensäure (85 Masse-%) werden in ein Reaktionsgefäß überführt und auf 100 °C erwärmt. Nach Erreichen der Reaktionstemperatur wird das Reaktionsgefäß geschüttelt (305 Schüttelbewegungen pro Minute) bis die Pinkfarbe der Nitrosoverbindung verschwindet. Die bis dahin erforderliche Zeit ist ein Maß für die Wirksamkeit des Katalysators und damit auch für die Effektivität des Hydrierverfahrens.

Die Reaktionszeiten bis zum vollständigen Umsatz des 1,3-Dimethyl-4-Amino-5-Nitrosouracil sind in der Tabelle 2 zusammengestellt.

**Tabelle 2**

| Katalysator | Reaktionszeit bis zum vollständigen Umsatz in min |
|---|---|
| A | 176 |
| B | 330 |
| D | 36 |
| E | 32 |

Die Meßdaten der Tabelle 2 belegen den Vorzug der Erfindung.

## Patentansprüche

1. Kohlenstoffhaltige Katalysatorträger für Katalysatoren zur Herstellung von Feinchemikalien, bestehend aus einem anorganischen Träger mit definierter Porenstruktur, wobei der Anteil des Porenvolumens mit Porenradien kleiner als 2 nm am Gesamtporenvolumen unterhalb von 5% liegt, und Oberflächen von 1 bis 900 m²/g, dessen Oberfläche mit polymeren und/oder polykondensierten Kohlenstoffverbindungen überzogen ist, wobei der Kohlenstoffgehalt 0,1 bis 30 Masse-%, bezogen auf den Katalysatorträger, beträgt, wobei die die Oberfläche überziehenden polymeren und/oder polykondensierten Kohlenstoffverbindungen herstellbar sind durch thermisches Zersetzen eines Gemisches ausgewählt aus der Gruppe bestehend aus a) Anilin und Cyclohexen, b) Phenol und Cyclohexanon, c) Methylcyclopentan und Benzoesäure, d) Butenen und Phenolen, e) 5 Masse-% o-Xylol und 95 Masse-% Phenol, f) 0,6 g/h Methylcyclopentan, 1,9 g/h Ethanol und 2,1 g/h Benzoesäure, g) 5 Masse-% m-Xylol, 30 Masse-% Cyclohexanon und 65 Masse-% Phenol und h) 3,2 g/h Cyclohexen, 2,6 g/h Cyclohexanon und 3,3 g/h Phenol im Stickstoffstrom an einer anorganischen Trägeroberfläche oder durch Verkoken des anorganischen Trägers mit Methylcyclopentan und nachfolgender Behandlung mit H₂O₂, NaOCl oder anderen oxidierend wirkenden Reagenzien und wobei die polymeren und/oder polykondensierten Kohlenstoffverbindungen Ionentauschereigenschaften aufweisen.

2. Katalysatorträger nach Anspruch 1, die als anorganische Trägermaterialien Al₂O₃, SiO₂, TiO₂, SiO₂ · Al₂O₃, Tone, Zeolithe, ZrO₂ oder natürliche Silicate und/oder beliebige Gemische dieser Träger mit Oberflächen von 100 bis 350 m²/g enthalten.

3. Katalysatorträger nach den Ansprüchen 1 und 2, mit einem Kohlenstoffgehalt von 5 bis 20 Masse-%, bezogen auf den Katalysatorträger.

4. Katalysatorträger nach einem der Ansprüche 1 bis 3, wobei der anorganische Träger durch Mischen verschiedener oder gleicher Trägermaterialien unterschiedlicher Textur hergestellt ist.

5. Verfahren zur Herstellung von funktionellen Gruppen aufweisenden kohlenstoffhaltigen Katalysatorträgern nach einem der Ansprüche 1 bis 4, **dadurch gekennzeichnet, daß** ein Gemisch ausgewählt aus der Gruppe bestehend aus a) Anilin und Cyclohexen, b) Phenol und Cyclohexanon, c) Methylcyclopentan und Benzoesäure, d) Butenen und Phenolen, e) 5 Masse-% o-Xylol und 95 Masse-% Phenol, f) 0,6 g/h Methylcyclopentan, 1,9 g/h Ethanol und 2,1 g/h Benzoesäure, g) 5 Masse-% m-Xylol, 30 Masse-% Cyclohexanon und 65 Masse-% Phenol und h) 3,2 g/h Cyclohexen, 2,6 g/h Cyclohexanon und 3,3 g/h Phenol im Stickstoffstrom an der Oberfläche eines anorganischen Trägers thermisch zersetzt oder der anorganische Träger mit Methylcyclopentan verkokt und anschließend mit H₂O₂, NaOCl oder anderen oxidierend wirkenden Reagenzien behandelt wird, so daß ein mit polymeren und/oder polykondensierten Kohlenstoffverbindungen überzogener Katalysatorträger entsteht.

6. Verwendung eines Katalysatorträgers nach den Ansprüchen 1 bis 4 für einen Pd-haltigen Katalysator mit und ohne Promotoren, wobei als Promotor die Metalle der 6., 7. und 8. Nebengruppe, vorzugsweise Pt, eingesetzt werden, zur Hydrierung von Nitroaromaten, **dadurch gekennzeichnet, daß** für die Reaktion ein Katalysator eingesetzt wird, der 0,1 bis 15 Masse-% Pd, vorzugsweise 1 bis 4 Masse-% Pd, auf einem Träger enthält, dessen anorganische Grundsubstanz durch Umsetzen von organischen Verbindungen mit funktionellen Gruppen in nichtoxidierender Atmosphäre mit einer funktionalisierten Kohlenstoffschicht bedeckt ist, mit
a) einem Anteil der Palladiumoberflächenatome an der Gesamtzahl der Palladiumatome von mindestens 50 %, vorzugsweise mindestens 80 %,
b) einem Kohlenstoffgehalt des Trägers von 1 bis 20 Masse-%, vorzugsweise 4 bis 10 Masse-%, und
c) einem Anteil des Porenvolumens mit Porenradien < 2 nm am Gesamtporenvolumen geringer als 5 %.

7. Verwendung nach Anspruch 6, **dadurch gekennzeichnet, daß** der Träger für einen Katalysator mit einer definierten Korngrößenverteilung von 1 bis 100 µm, vorzugsweise von 5 bis 20 µm, eingesetzt wird.

8. Verwendung eines Katalysatorträgers nach den Ansprüchen 1 bis 4 für einen Pd-haltigen Katalysator zur Hydrierung von 1,3-Dimethyl-4-Amino-5-Nitrosouracil mit gasförmigem Wasserstoff oder mit Wasserstoff, der durch katalytischen Zerfall von Ameisensäure gebildet wird, bei Temperaturen von 20 bis 120°C und Drücken von 0,1 bis 10 MPa, **dadurch gekennzeichnet, daß** für die Reaktion ein Katalysator eingesetzt wird, der 0,5 bis 7 Masse-% Pd, vorzugsweise 2 bis 4 Masse-% Pd, auf einem Träger enthält, dessen anorganische Grundsubstanz durch Umsetzen organischer Verbindungen in nichtoxidierender Atmosphäre mit einer funktionalisierten Kohlenstoffschicht bedeckt ist, mit
a) einem Anteil der Palladiumoberflächenatome an der Gesamtzahl der Palladiumatome von mindestens 75 %, vorzugsweise 90 %,
b) einem Kohlenstoffgehalt des Trägers von 1 bis 15 Masse-%, vorzugsweise 3 bis 8 Masse-%, und
c) einem Anteil des Porenvolumens mit Porenradien < 1,5 nm am Gesamtporenvolumen geringer als 10 %.

## Claims

1. Carbon-containing catalyst support for catalysts for the manufacture of fine chemicals, consisting of an inorganic support of defined pore structure, wherein the proportion of the pore volume with pore radii smaller than 2nm is below 5% of the total pore volume, and surface areas of 1 to 900 m²/g, of which the surface is coated with polymer and/or poly-condensed carbon compounds, wherein the carbon content is 0.1% to 30% by mass, relative to the catalyst support, wherein the polymer and/or poly-condensed carbon compounds coating the surface can be manufactured by thermal decomposition of the mixture selected from the group consisting of a) aniline and cyclohexene, b) phenol and cyclohexanone, c) methylcyclopentane and benzoic acid, d) butenes and phenols, e) 5% by mass o-xylene and 95% by mass phenol, f) 0.6 g/h methylcyclopentane, 1.9 g/h ethanol and 2.1 g/h benzoic acid, g) 5% by mass m-xylene, 30% by mass cyclohexanone and 65% by mass phenol and h) 3.2 g/h cyclohexene, 2.6 g/h cyclohexanone and 3.3 g/h phenol in the nitrogen stream on an inorganic support surface or by coking of the inorganic support with methylcyclopentane and subsequent treatment with H₂O₂, NaOCl or other oxidative reagents, and wherein the polymer and/or poly-condensed carbon compounds provide ionic exchange properties.

2. Catalyst supports according to claim 1, which contain as the inorganic support materials Al₂O₃, SiO₂, TiO₂, SiO₂, Al₂O₃, clays, zeolites, ZrO₂ or natural silicates and/or any other mixtures of these supports with surface areas from 100 to 350 m²/g.

3. Catalyst supports according to claims 1 and 2, with a carbon content of 5% to 20% by mass relative to the catalyst support.

4. Catalyst supports according to any one of claims 1 to 3, wherein the inorganic support is manufactured by mixing either different support materials or the same support materials with a different texture.

5. Method for manufacturing carbon-containing catalyst supports providing functional groups according to any one of claims 1 to 4, **characterised in that** a mixture selected from the group consisting of a) aniline and cyclohexene, b) phenol and cyclohexanone, c) methylcyclopentane and benzoic acid, d) butenes and phenols, e) 5% by mass o-xylene and 95% by mass phenol, f) 0.6 g/h methylcyclopentane, 1.9 g/h ethanol and 2.1 g/h benzoic acid, g) 5% by mass m-xylene, 30% by mass cyclohexanone and 65% by mass phenol and h) 3.2 g/h cyclohexene, 2.6 g/h cyclohexanone and 3.3 g/h phenol undergoes thermal decomposition in the nitrogen stream on an inorganic support surface or that the inorganic support is coked with methylcyclopentane and subsequently treated with H₂O₂, NaOCl or other oxidative reagents, so that a catalyst support coated with the polymer and/or poly-condensed carbon compounds provided.

6. Use of a catalyst support according to claims 1 to 4 for a Pd-containing catalyst with or without promoters, wherein the metals of the 6^{th}, 7^{th} and 8^{th} subgroup, by preference Pt, are used as the promoter, for the hydration of nitro-aromatics, **characterised in that** a catalyst is used for the reaction, which contains 0.1% to 15% by mass Pd, by preference 1% to 4% by mass Pd on a support, of which the inorganic base substance is coated by conversion of organic compounds with functional groups in a non-oxidising atmosphere with a functionalised carbon layer, with
a) a proportion of the Palladium surface atoms of at least 50%, by preference at least 80%, of the total number of Palladium atoms,
b) a carbon content of the support from 1% to 20% by mass, preferably 4% to 10 % by mass, and
c) a proportion of the pore volume with pore radii < 2 nm comprising less than 5% of the total pore volume.

7. Use according to claim 6, **characterised in that** the support is used for a catalyst with a defined grain size distribution from 1 to 100 µm, by preference, from 5 to 20 µm.

8. Use of a catalyst support according to claims 1 to 4 for a Pd-containing catalyst for hydration of 1,3-dimethyl-4-amino-5-nitrosouracil with gaseous hydrogen or with hydrogen which is formed by catalytic degradation of formic acid, at temperatures from 20 to 120°C and pressures from 0.1 to 10 MPa, **characterised in that** a catalyst is used for the reaction which contains 0.5% to 7% by mass Pd, by preference 2% to 4% by mass Pd on a support, of which the inorganic base substance is coated by conversion of organic compounds in a non-oxidising atmosphere with a functionalised carbon layer, with
a) a proportion of the Palladium surface atoms of at least 75%, by preference at least 90%, of the total number of Palladium atoms,
b) a carbon content of the support from 1% to 15% by mass, preferably 3% to 8 % by mass, and
c) a proportion of the pore volume with pore radii < 1.5 nm comprising less than 10% of the total pore volume.

## Revendications

1. Supports de catalyseur contenant du carbone pour catalyseurs utilisés pour la préparation de produits chimiques fins, constitués d'un support inorganique comportant une structure poreuse définie, le rapport du volume de pores avec des rayons de pores inférieurs à 2 nm au volume de pores total étant inférieur à 5%, et des surfaces de 1 à 900 m²/g, dont la surface est recouverte de composés carbonés polymères et/ou polycondensés, la teneur en carbone étant de 0,1 à 30 % en masse, par rapport au support de catalyseur, les composés carbonés polymères et/ou polycondensés recouvrant la surface pouvant être préparés par décomposition thermique d'un mélange choisi dans le groupe constitué de a) l'aniline et le cyclohexène, b) le phénol et le cyclohexanone, c) le méthylcyclopentane et l'acide benzoïque, d) les butènes et les phénols, e) 5% en masse de o-xylène et 95 % en masse de phénol, f) 0,6 g/h de méthylcyclopentane, 1,9 g/h d'éthanol et 2,1 g/h d'acide benzoïque, g) 5 % en masse de m-xylène, 30 % en masse de cyclohexanone et 65 % en masse de phénol et h) 3,2 g/h de cyclohexène, 2,6 g/h de cyclohexanone et 3,3 g/h de phénol, dans un courant d'azote sur une surface support inorganique ou par cokéfaction du support inorganique avec du méthylcyclopentane et traitement subséquent avec H₂O₂, NaOCl ou d'autres réactifs oxydants, les composés carbonés polymères et/ou polycondensés présentant des propriétés d'échangeurs d'ions.

2. Supports de catalyseurs selon la revendication 1, qui contiennent comme matières supports inorganiques Al₂O₃, SiO₂, TiO₂, SiO₂.Al₂O₃, les argiles, les zéolithes, ZrO₂ ou les silicates naturels et/ou des mélanges quelconques de ces supports avec des surfaces de 100 à 350 m²/g.

3. Supports de catalyseur selon les revendications 1 et 2, d'une teneur en carbone de 5 à 20 % en masse par rapport au support de catalyseur.

4. Supports de catalyseur selon l'une quelconque des revendications 1 à 3, le support inorganique étant fabriqué par mélange de matières supports identiques ou différentes de texture différente.

5. Procédé pour la préparation de supports de catalyseurs contenant du carbone et présentant des groupes fonctionnels selon l'une quelconque des revendications 1 à 4, **caractérisé en ce qu'**un mélange choisi dans le groupe constitué de a) l'aniline et le cyclohexène, b) le phénol et le cyclohexanone, c) le méthylcyclopentane et l'acide benzoïque, d) les butènes et les phénols, e) 5% en masse de o-xylène et 95 % en masse de phénol, f) 0,6 g/h de méthylcyclopentane, 1,9 g/h d'éthanol et 2,1 g/h d'acide benzoïque, g) 5 % en masse de m-xylène, 30 % en masse de cyclohexanone et 65 % en masse de phénol et h) 3,2 g/h de cyclohexène, 2,6 g/h de cyclohexanone et 3,3 g/h de phénol, est décomposé par voie thermique dans un courant d'azote à la surface d'un support inorganique ou le support inorganique est cokéfié avec du méthylcyclopentane et ensuite traité avec H₂O₂, NaOCl ou d'autres réactifs oxydants, de telle sorte qu'il se forme un support de catalyseur recouvert de composés carbonés polymères et/ou polycondensés.

6. Utilisation d'un support de catalyseur selon les revendications 1 à 4 pour un catalyseur contenant du Pd avec et sans promoteurs, les métaux des Groupes 6A, 7A et 8, de préférence Pt, étant utilisés comme promoteurs, pour l'hydrogénation de composés nitro-aromatiques, **caractérisée en ce qu'**on utilise pour la réaction un catalyseur qui contient 0,1 à 15 % en masse de Pd, de préférence 1 à 4 % en masse de Pd, sur un support dont la substance de base inorganique est recouverte d'une couche carbonée fonctionnalisée par transformation de composés organiques ayant des groupes fonctionnels en atmosphère non oxydante, avec
a) un rapport des atomes superficiels de palladium au nombre total d'atomes de palladium d'au moins 50%, de préférence d'au moins 80%,
b) une teneur en carbone du support de 1 à 20 % en masse, de préférence 4 à 10 % en masse, et
c) un rapport du volume de pores avec des rayons de pores < 2nm au volume de pores total inférieur à 5%.

7. Utilisation selon la revendication 6, **caractérisée en ce que** le support est utilisé pour un catalyseur présentant une distribution granulométrique définie de 1 à 100 µm, de préférence de 5 à 20 µm.

8. Utilisation d'un support de catalyseur selon les revendications 1 à 4 pour un catalyseur contenant du Pd pour l'hydrogénation de 1,3-diméthyl-4-amino-5-nitroso-uracile avec de l'hydrogène gazeux ou avec de l'hydrogène qui est formé par décomposition catalytique d'acide formique, à des températures de 20 à 120°C et sous des pressions de 0,1 à 10 MPa, **caractérisée en ce qu'**on utilise pour la réaction un catalyseur qui contient 0,5 à 7% en masse de Pd, de préférence 2 à 4 % en masse de Pd, sur un support dont la substance de base inorganique est recouverte d'une couche carbonée fonctionnalisée par transformation de composes organiques en atmosphère non oxydante, avec
a) un rapport des atomes superficiels de palladium au nombre total d'atomes de palladium d'au moins 75 %, de préférence 90%,
b) une teneur en carbone du support de 1 à 15 % en masse, de préférence 3 à 8 % en masse, et
c) un rapport du volume de pores avec des rayons de pores < 1,5 nm au volume de pores total inférieur à 10 %.
